# EUROPÄISCHE PATENTSCHRIFT

(11) **EP 4 103 105 B1**
(45) Veröffentlichungstag und Bekanntmachung des Hinweises auf die Patenterteilung: **26.07.2023**
(21) Anmeldenummer: 21712846.1
(22) Anmeldetag: 22.03.2021
(51) Int. Cl.: A61F 2/01, A61F 2/95, A61B 17/12, A61B 17/00

(54) **SYSTEM ZUR IMPLANTATION EINES MEDIZINISCHEN IMPLANTATS IM MENSCHLICHEN ODER TIERISCHEN KÖRPER**
SYSTEM FOR THE IMPLANTATION OF A MEDICAL IMPLANT IN THE HUMAN OR ANIMAL BODY
SYSTÈME POUR L'IMPLANTATION D'UN IMPLANT MÉDICAL DANS LE CORPS HUMAIN OU ANIMAL

(30) Priorität: 25.03.2020 IT 202000006286
(43) Veröffentlichungstag der Anmeldung: 21.12.2022
(73) Patentinhaber: PFM Medical AG, 50996 Köln (DE)
(72) Erfinder: SCHÄFER, Joachim, 66620 Nonnweiler (DE)
(74) Vertreter: Hohendorf Kierdorf Patentanwälte PartGmbB
(86) Internationale Anmeldenummer: PCT/EP2021/057223
(87) Internationale Veröffentlichungsnummer: WO 2021/191129

(56) Entgegenhaltungen:
- EP-A1- 0 880 341
- EP-A2- 0 684 022
- WO-A1-2019/105073
- US-A1- 2002 123 698
- US-A1- 2010 268 201

## Beschreibung

Die Erfindung betrifft ein System zur Implantation eines medizinischen Implantats im menschlichen oder tierischen Körper. Ein medizinisches Implantat ist eine im menschlichen oder tierischen Körper eingepflanzte, künstliche Vorrichtung, welche permanent oder temporär innerhalb des menschlichen oder tierischen Körpers verbleibt.

Ein Beispiel für ein medizinisches Implantat ist ein sogenannter Stent, welcher in Hohlorgane des menschlichen oder tierischen Körpers eingebracht wird, um diese offen zu halten. Bei einem Stent kann es sich beispielsweise um ein kleines Gittergerüst in Röhrenform aus Metall oder Kunstfasern handeln. Verwendung finden Stents zum einen in Blutgefäßen, speziell in den Herzkranzgefäßen, um nach deren Aufdehnung einen erneuten Verschluss zu verhindern. Zum anderen dienen Stents in der Krebsbehandlung dazu, durch bösartige Tumore verursachte Verengungen nach einer Aufdehnung offen zu halten.

Weiterhin finden medizinische Implantate in der Herzchirurgie Anwendung, wobei die medizinischen Implantate meist dazu dienen, eine abnormale Öffnung innerhalb des Herzens zu verschließen. Beispiele für derartige Anwendungen sind die Behandlung von Ventrikel Septum Defekten (VSD), Persistierenden Foramen Ovale (PFO), Persistierender Ductus Arteriosus (PDA), Atrium Septum Defekten (ASD) oder der Verschluss des linken Herzohrs (Left Atrial Appendage, LAA).

Soweit möglich werden derartige medizinische Implantate mittels eines minimalinvasiven Verfahrens in den menschlichen oder tierischen Körper eingebracht. Bei einem minimalinvasiven Verfahren wird ein Zugang zu einem großen Blutgefäß innerhalb des menschlichen oder tierischen Körpers geschaffen, über welchen ein Katheter in den menschlichen oder tierischen Körper eingeführt wird. Über die Blutgefäße wird dieser Katheter zur Implantationsstelle geführt. Nachfolgend wir über den Katheter das medizinische Implantat mittels einer Einführhilfe transportiert.

Üblicherweise bestehen die medizinischen Implantate aus einem Formgedächtnismaterial, so dass sich das medizinische Implantat nach Verlassen des Katheters in eine vorgegebene Form entfaltet. Das medizinische Implantat wird also mittels der Einführhilfe über den Katheter zu der Implantationsstelle transportiert und entfaltet sich nach Verlassen des Katheters in die vorgegebene Form. Nachdem das medizinische Implantat korrekt an der Implantationsstelle in den menschlichen oder tierischen Körper eingesetzt wurde, kann die Verbindung zwischen Einführhilfe und medizinischen Implantat gelöst werden.

Folglich muss das medizinische Implantat während des Transports durch den Katheter möglichst fest mit der Einführhilfe verbunden sein und sich gleichzeitig nach erfolgter Implantation einfach von der Einführhilfe lösen lassen. Aus dem Stand der Technik ist es beispielsweise bekannt, das medizinische Implantat auf einen Trägerdraht der Einführhilfe aufzuklemmen und die Klemmverbindung nach erfolgreicher Implantation des medizinischen Implantats zu lösen. Nachteilig an einer derartigen Verbindung zwischen dem medizinischen Implantat und der Einführhilfe ist jedoch, dass zum Lösen der Verbindung zwischen Implantat und Trägerdraht eine relativ hohe Kraft notwendig ist und weiterhin kann die Verbindung zum medizinischen Implantat und Trägerdraht, welcher auf einer Reibkraft basiert, beispielsweise durch Flüssigkeiten negativ beeinflusst werden, so dass sich die Verbindung zwischen medizinischen Implantat und Trägerdraht frühzeitig lösen kann.

Die WO 2015/078807 A1 offenbart ein System zum Verbinden eines medizinischen Implantats mit einer Einführhilfe, wobei das medizinische Implantat und die Einführhilfe jeweils einen spiralförmigen Verbindungsbereich aufweisen, welche im verbundenen zustand ineinandergreifen und durch einen Riegeldraht miteinander relativ zueinander fixiert sind. Dies setzt jedoch einen speziell ausgestalteten spiralförmigen Verbindungsbereich am medizinischen Implantat voraus.

Die US 2010/0268201 A1 offenbart ein weiteres System zum Implantieren eines medizinischen Implantats.

Der Erfindung liegt somit die Aufgabe zugrunde, ein System zur Implantation eines medizinischen Implantats im menschlichen oder tierischen Körper bereitzustellen, welches während der Implantation des medizinischen Implantats im menschlichen oder tierischen Körper eine sichere Verbindung zwischen medizinischen Implantat und Einführhilfe gewährleistet und gleichzeitig ein einfaches Lösen der Verbindung zwischen medizinischen Implantat und Einführhilfe nach erfolgter Implantation des medizinischen Implantats ermöglicht. Ferner sollte die Verbindung zwischen dem medizinischen Implantat und der Einführhilfe aufgrund eines Einsatzes in der minimalinvasiven Chirurgie einen möglichst geringen Durchmesser aufweisen, vorzugsweise einen Durchmesser der kleiner oder gleich dem Durchmesser des medizinischen Implantats bzw. der Einführhilfe ist.

Die Aufgabe wird erfindungsgemäß gelöst durch ein System zur Implantation eines medizinischen Implantats im menschlichen oder tierischen Körper, umfassend:
ein drahtartiges Element zur Verbindung des Systems mit dem medizinischen Implantat,
einen doppellumigen Führungsschlauch für das drahtartige Element, und
einen Handgriff zur Handhabung des Systems,
wobei das medizinische Implantat am proximalen Ende des Führungsschlauchs angeordnet ist und der Handgriff am distalen Ende des Führungsschlauchs, und wobei das drahtartige Element mit einem ersten Ende am Handgriff fixiert ist, durch ein erstes Lumen des doppellumigen Führungsschlauchs zu dem medizinischen Implantat geführt ist, mit einer Verbindungseinrichtung des medizinischen Implantat eingreift und durch ein zweites Lumen des doppellumigen Führungsschlauchs zum Handgriff geführt ist und am Handgriff mit einem zweiten Ende lösbar fixiert ist.

Bei der Herstellung des erfindungsgemäßen Systems wird beispielsweise das drahtartige Element durch eine Verbindungseinrichtung wie eine Schlaufe oder Öse des medizinischen Implantats geführt, um eine lösbare Verbindung zwischen dem erfindungsgemäßen System und dem medizinischen Implantat herzustellen. Ein erstes Ende des drahtartigen Elements wird nachfolgend durch das erste Lumen des doppellumigen Führungsschlauchs geführt und das zweite Ende des drahtartigen Elements wird durch das zweite Lumen des doppellumigen Führungsschlauchs geführt. Dadurch wird das medizinische Implantat am proximalen Ende des doppellumigen Führungsschlauchs fixiert. Am distalen Ende des doppellumigen Führungsschlauchs wird ein Ende des drahtartigen Elements an dem Handgriff fixiert, während das andere Ende des drahtartigen Elements lösbar mit dem Handgriff verbunden wird. Mittels des so hergestellten Systems kann das medizinische Implantat über einen bereits zur Implantationsstelle im menschlichen oder tierischen Körper gelegten Katheter implantiert werden. Nachdem das medizinische Implantat korrekt im menschlichen Körper positioniert wurde, was beispielsweise mittels eines bildgebenden Verfahrens überprüft wird, wird die Verbindung zwischen dem erfindungsgemäßen System und dem medizinischen Implantat gelöst, so dass das medizinische Implantat im menschlichen oder tierischen Körper verbleibt und das erfindungsgemäße System wieder entfernt werden kann. Dazu wird die lösbare Verbindung zwischen Handgriff und einem Ende es drahtartigen Elements gelöst. Durch Ziehen an dem Handgriff wird das drahtartige Element durch die Fixierung des anderen Endes des drahtartigen Elements an dem Handgriff aus dem menschlichen oder tierischen Körper gezogen. Dadurch wird gleichzeitig das drahtartige aus der Schlaufe oder Öse des medizinischen Implantats gezogen, wodurch die Verbindung zwischen dem erfindungsgemäßen System und dem medizinischen Implantat gelöst wird. Nach dem Lösen dieser Verbindung kann der doppellumige Schlauch aus dem menschlichen oder tierischen Körper entfernt werden.

Das proximale Ende des doppellumigen Führungsschlauchs im Sinne der Erfindung ist das dem menschlichen oder tierischen Körper zugewandte Ende und das distale Ende des doppellumigen Führungsschlauchs im Sinne der Erfindung ist das dem menschlichen Körper abgewandte Ende.

Die Verbindung zwischen dem erfindungsgemäßen System und dem medizinischen Implantat weist einen sehr geringen Durchmesser auf, da lediglich das drahtartige Element durch eine öse oder Schlaufe des medizinischen Implantats geführt werden muss. Es wird kein zusätzlicher Riegeldraht oder ähnliches benötigt und auch keine speziell ausgeformten Verbindungsabschnitte am medizinischen Implantat und/oder der Einführhilfe wie im Stand der Technik offenbart. Somit kann der für die Implantation benötigte Durchmesser des Katheters reduziert werden.

Der doppellumige Führungsschlauch verhindert dabei, dass sich das drahtartige Element ineinander verdreht, wodurch das Entfernen und somit Lösen des medizinischen Implantats deutlich erschwert würde. Durch jedes Lumen des doppellumigen Führungsschlauch ist jeweils nur ein Abschnitt des drahtartigen Elements geführt.

Das erfindungsgemäße System umfasst eine sehr geringe Anzahl von Komponenten, welche gleichzeitig einen möglichst einfachen Aufbau haben. Dadurch lässt sich das erfindungsgemäße System einfach und kostengünstig herstellen. Ferner verringern sich Lager-, Verpackungs- und Transportkosten. Auch ist die Handhabung sehr einfach und intuitiv und der Anwender muss keine besonderen Kenntnisse für die Nutzung haben.

Gemäß einer vorteilhaften Variante der Erfindung besteht das drahtartige Element aus einem Metall, insbesondere aus Edelstahl. Ein drahtartiges Element aus Metall weist eine hohe Zugfestigkeit bei einem geringen Durchmesser auf, wodurch der Durchmesser des erfindungsgemäßen Systems minimiert werden kann. Dies wirkt sich Positiv auf die Implantation aus, insbesondere da ein Katheter mit einem geringeren Durchmesser für die Implantation benutzt werden kann.

Nach einer zweckmäßigen Variante der Erfindung weist das drahtartige Element einen Durchmesser von 0,1 mm bis 0,5 mm auf, insbesondere einen Durchmesser von 0,2 mm.

In einer bevorzugten Variante der Erfindung besteht der doppellumige Führungsschlauch zumindest teilwiese aus einem Kunststoff. Dadurch ist der doppellumige Führungsschlauch flexibel, was die Führung des Führungsschlauchs durch das Gefäßsystem des menschlichen oder tierischen Körpers vereinfacht. Ein flexibel Führungsschlauchs passt sich beispielsweise der Form eines bereits gelegten Katheters an. Die mechanischen Eigenschaften des doppellumigen Führungsschlauchs können ferner durch Verstärkungselemente wie in dem Kunststoff integrierte Metallelement, insbesondere Drähte oder Metallgitter, angepasst werden. Auch kann der Führungsschlauch durch in den Kunststoff eingebrachte Drähte steuerbar ausgebildet werden.

Nach einer zweckmäßigen Variante der Erfindung weist der doppellumige Führungsschlauch einen Durchmesser von 1,0 mm bis 2,0 mm auf, insbesondere einen Durchmesser von 1,76 mm.

Die einzelnen Lumen des doppellumigen Führungsschlauch weisen einen an den Durchmesser des drahtartigen Elements angepassten Innendurchmesser auf, so dass das drahtartige Element durch die Lumen des doppellumigen Führungsschlauchs geführt werden kann. Insbesondere ist der Durchmesser der Lumen wenigstens geringfügig größer als der Durchmesser des drahtartigen Elements.

Gemäß einer Variante der Erfindung umfasst der doppellumige Führungsschlauch zwei innenliegende schlauchartige Elemente, welche das erste Lumen und das zweite Lumen des doppellumigen Führungsschlauchs bilden. Der doppellumige Führungsschlauch besteht also aus insgesamt drei schlauchartigen Elementen, wobei zwei schlauchartige Elemente in einem äußeren schlauchartigen Element angeordnet sind. Die zwei innenliegenden schlauchartigen Elemente bilden dabei das erste Lumen und das zweite Lumen des doppellumigen Führungsschlauchs.

Die Erfindung ist auch nicht auf einen doppellumigen Führungsschlauch begrenzt, sondern kann auch mit einem mehrlumigen Schlauch verwirklicht werden. Für die Führung des drahtartigen Elements werden jedoch nur zwei Lumen benötigt. Eventuell weitere Lumen können für andere Zwecke genutzt werden, wie beispielsweise Injektion von Flüssigkeiten wie Kontrastmittel für bildgebende Verfahren oder das Injizieren oder Einführen anderer Flüssigkeiten, Gegenstände und/oder Gerätschaften.

In einer zweckmäßigen Variante der Erfindung sind die zwei innenliegenden schlauchartige Elemente jeweils als Edelstahlwende ausgebildet. Derartige Edelstahlwendel weisen eine hohe radiale Flexibilität auf, bei einer gleichzeitigen Stabilität in Längsrichtung.

Nach einer vorteilhaften Variante der Erfindung ist die Verbindungseinrichtung des medizinischen Implantats als Öse oder Schlaufe ausgebildet. Eine derartige Öse oder Schlaufe lässt sich einfach an dem medizinischen Implantat herstellen, da medizinische Implantate häufig aus drahtartigen Elementen hergestellt werden. Es muss als nur aus dem drahtartigen Element des medizinischen Implantats eine Öse oder Schlaufe geformt werden, beispielsweise an einem Ende des medizinischen Implantats.

Gemäß einer weiteren Variante der Erfindung umfasst das erste Ende des drahtartigen Elements einen Nippel, welcher in einer entsprechenden Ausnehmung des Handgriffs fixierbar ist. Dadurch lässt sich das erste Ende des drahtartigen Elements einfach am Handgriff fixieren. Es sind aber auch andere Fixierungen möglich, wie Kleben, Schweißen, Löten und dergleichen.

Erfindungsgemäß umfasst der Handgriff einen Klemmmechanismus für das zweite Ende des drahtartigen Elements, zur lösbaren Fixierung des zweiten Endes des drahtartigen Elements am Handgriff. Durch den Klemmmechanismus lässt sich das zweite Ende lösbar am Handgriff fixieren. Nach erfolgreicher Implantation des medizinischen Implantats wird die Klemmverbindung gelöst und die Verbindung zwischen dem erfindungsgemäßen System und dem medizinischen Implantat kann durch Herauszeihen des drahtartigen Elements aus der Öse oder Schlaufe des medizinischen Implantats gelöst werden. Dies wird dadurch erreicht, dass das erste Ende des drahtartigen Elements am Handgriff fixiert ist und die Verbindung zum zweiten Ende des drahtartigen Elements gelöst wird und das drahtartige Element mittels des Handgriffs gezogen wird.

Nach der Erfindung umfasst der Klemmmechanismus eine Sicherung gegen ein unbeabsichtigtes Betätigen des Klemmmechanismus, insbesondere gegen ein unbeabsichtigtes Lösen des Klemmmechanismus. Es muss also vor einer Betätigung des Klemmmechanismus die Sicherung gelöst werden.

Gemäß einer weiteren zweckmäßigen Variante der Erfindung umfasst das erfindungsgemäße System einen Implantationskatheter zur Führung des doppellumigen Führungsschlauchs und des damit verbundenen medizinischen Implantats zur Implantationsstelle im menschlichen oder tierischen Körper. Der Implantationskatheter wird durch das Gefäßsystem des menschlichen oder tierischen Körpers zur Implantationsstelle geführt. Dazu wird beispielsweis ein Zugang zu einem größeren Gefäß des menschlichen oder tierischen Körpers hergestellt, insbesondere in der Leistengegend des menschlichen Körpers. Ein steuerbarer Katheter kann durch das Gefäßsystem zur Implantationsstelle im menschlichen oder tierischen Körper gelenkt werden. Alternativ wird der Implantationskatheter mittels eines bereits gelegten Führungsdrahts zur Implantationsstelle geführt. Über den Implantationskatheter wird das medizinische Implantat mittels des doppellumigen Führungsschlauchs durch den Implantationskatheter vorgeschoben. Der doppellumige Führungsschlauch übernimmt dabei die Funktion eines sogenannten Pushers, welcher das medizinische Implantat durch den Implantationskatheter zur Implantationsstelle vorschiebt. Nach erfolgreicher Implantation wird die Verbindung des erfindungsgemäßen Systems zum medizinischen Implantat wie vorbeschrieben gelöst und der doppellumige Führungsschlauch und der Implantationskatheter können aus dem menschlichen oder tierischen Körper entfernt werden.

In einer Variante der Erfindung umfasst das System zusätzlich einen Y-Konnektor zum Spülen des Systems, insbesondere des doppellumigen Führungsschlauchs und/oder des Implantationskatheters.

Nach einer weiteren Variante umfasst das System ferner einen sogenannten Pusher, um den doppellumigen Führungsschlauch und das damit verbundene medizinische Implantat durch den Implantationskatheter zu bewegen.

Nachfolgend wird die Erfindung anhand der in den Figuren dargestellten Ausführungsbeispiele näher erläutert. Es zeigen:
- Fig. 1: eine Ansicht eines erfindungsgemäßen Systems,
- Fig. 2: eine Detailansicht des proximalen Endes des Systems aus Figur 1,
- Fig. 3: eine Detailansicht eines Handgriffs eines erfindungsgemäßen Systems,
- Fig. 4: eine Detailansicht eines mit einem erfindungsgemäßen System verbundenen medizinischen Implantats, und
- Fig. 5: eine Querschnittsansicht eines doppellumigen Führungsschlauchs eines erfindungsgemäßen Systems.

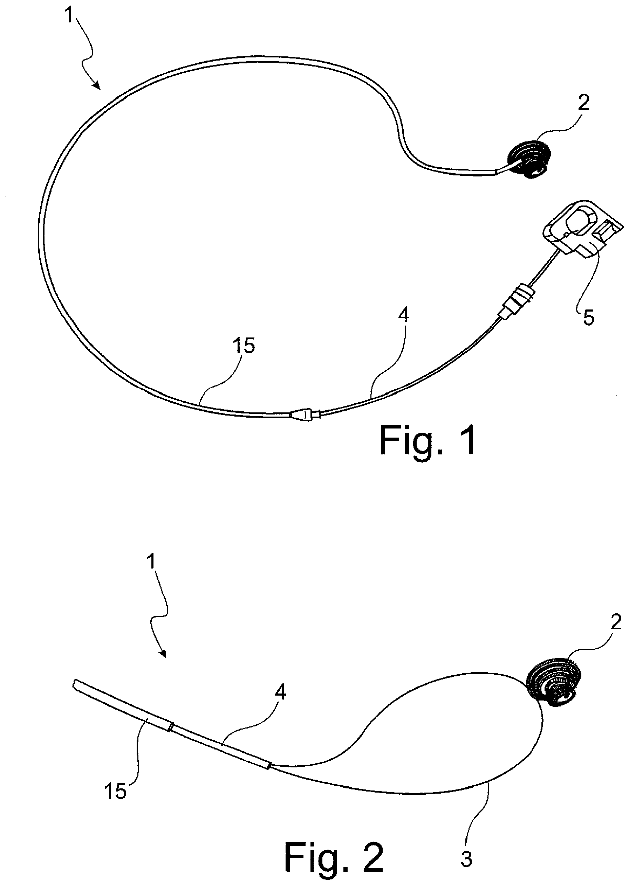

Figur 1 zeigt eine beispielhafte Ausführungsform eines erfindungsgemäßen Systems 1 zur Implantation eines medizinischen Implantats 2 im menschlichen oder tierischen Körper. Das System 1 umfasst ein drahtartiges Element 3 zur Verbindung des Systems 1 mit dem medizinischen Implantat 2. Das System 1 umfasst ferner einen doppellumigen Führungsschlauch 4 für das drahtartige Element 3 und einen Handgriff 5 zur Handhabung des Systems 1.

Das medizinische Implantat 2 ist am proximalen Ende des Führungsschlauchs 4 angeordnet und der Handgriff 5 am distalen Ende des Führungsschlauch 4, wobei proximal das dem menschlichen oder tierischen Körper zugewandte Ende des Führungsschlauchs 4 betrifft und distal das dem menschlichen oder tierischen Körper abgewandte Ende des Führungsschlauchs 4.

Das drahtartige Element 3 ist mit einem ersten Ende 9 am Handgriff 5 fixiert ist, durch ein erstes Lumen 6 des doppellumigen Führungsschlauchs 4 zu dem medizinischen Implantat 2 geführt, greift nachfolgend mit einer Verbindungseinrichtung 8 des medizinischen Implantats 2 ein und ist anschließend durch ein zweites Lumen 7 des doppellumigen Führungsschlauchs 4 zum Handgriff 5 geführt. Am Handgriff 5 ist das zweite Ende 10 des drahtartigen Elements 3 lösbar fixiert.

Gemäß dem Ausführungsbeispiel aus Fig. 1 umfasst das erfindungsgemäße System 1 ferner einen Implantationskatheter 15, mittels welchem das medizinische Implantat 2 mittels des doppellumigen Führungsschlauchs 4 zur Implantationsstelle im menschlichen oder tierischen Körper vorgeschoben wird.

Das drahtartige Element 3 besteht beispielsweise aus einem Metall, insbesondere Edelstahl und weist einen Durchmesser von 0,1 mm bis 0,5 mm, insbesondere 0,2 mm, auf.

Der doppellumige Führungsschlauch 4 besteht beispielsweise zumindest teilweise aus einem Kunststoff und weist einen Durchmesser von 1,0 mm bis 2,0 mm, insbesondere 1,76 mm, auf.

Die Verbindungseinrichtung 8 des medizinischen Implantats 2 ist vorzugsweise als Öse oder Schlaufe ausgebildet.

Zwischen dem Implantationskatheter 15 und dem Handgriff 5 ist ein sogenannter Pusher angeordnet, um den doppellumigen Führungsschlauch 4 und das darin befestigte medizinische Implantat 2 durch den Implantationskatheter 15 zu bewegen.

Ferner kann an dem Übergang zwischen Implantationskatheter 15 und doppellumigen Führungsschlauch 4 ein Y-Konnektor angeordnet werden, zum Spülen des erfindungsgemäßen System 1, insbesondere des Implantationskatheters 15.

Figur 2 zeigt eine Detailansicht der Verbindung des erfindungsgemäßen Systems 1 mit einem medizinischen Implantat 2 am proximalen Ende des Systems 1. Aus dem Implantationskatheter 15 tritt der doppellumige Führungsschlauch 4 mit dem daran befestigten medizinischen Implantat 2 aus. Das medizinische Implantat 2 ist mit dem erfindungsgemäßen System 1 durch das drahtartige Element 3 verbunden, wobei das drahtartige Element 2 in eine Verbindungseinrichtung 8 des medizinischen Implantats eingreift. Die Verbindungseinrichtung 8 des medizinischen Implantats ist beispielsweise als Öse oder Schlaufe ausgebildet. Auf dem Weg zum distalen Ende des erfindungsgemäßen Systems 1 sind die beiden Enden9, 10 des drahtartigen Elements durch das erste Lumen 6 und das zweite Lumen 7 des doppellumigen Führungsschlauchs 4 geführt.

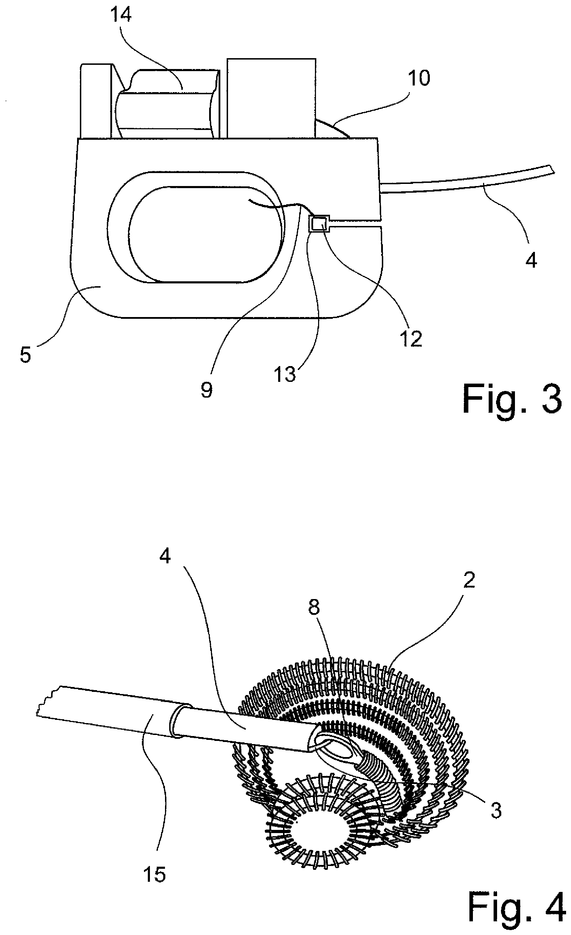

Figur 3 ist eine Detailansicht eines Handgriffs 5 eines erfindungsgemäßen Systems 1 zur Implantation eines medizinischen Implantats 2 im menschlichen oder tierischen Körper. Ein erstes Ende 9 des drahtartigen Elements 4 ist fest an dem Handgriff 5 fixiert. Dazu weist das drahtartige 4 an dem ersten beispielsweise einen Nippel 12 auf, welcher in eine entsprechende Ausnehmung 13 am Handgriff 5 eingreift. Es sind aber auch andere Fixierungen möglich, wie beispielsweise Kleben, Schweißen, Löten oder andere Verbindungstechniken. Das zweite Ende 10 des drahtartigen Elements 4 ist lösbar mittels eines Klemmmechanismus 14 mit dem Handgriff 5 verbunden.

Nach einer erfolgreichen Implantation des medizinischen Implantats 2 wird die Verbindung zwischen zweitem Ende 10 des drahtartigen Elements 4 und dem Handgriff 5 gelöst und das drahtartige Element 4 aus der Verbindungseinrichtung 8 des medizinischen Implantats 2 gezogen, um die Verbindung zwischen erfindungsgemäßen System 1 und medizinischem Implantat 2 zu lösen.

Nach der Erfindung umfasst der Klemmmechanismus 14 eine Sicherung (nicht dargestellt), welche ein unbeabsichtigtes Betätigen des Klemmmechanismus 14 verhindert. Die Sicherung ist beispielsweise als Riegel ausgebildet.

In Fig. 4 ist eine Detailansicht eines mit einem erfindungsgemäßen System 1 verbundenen medizinischen Implantats 2 dargestellt. Aus dem Implantationskatheter 15 tritt der doppellumige Führungsschlauch 4 aus. Durch das erste Lumen 6 des doppellumigen Führungsschlauchs 4 ist das drahtartige Element 3 vom Handgriff 5 des erfindungsgemäßen Systems 1 zu dem medizinischen Implantat 2 geführt. Das drahtartige Element 3 greift in die Verbindungseinrichtung 8 des medizinischen Implantats 2 ein und ist durch das zweite Lumen 7 zurück zum Handgriff 5 des erfindungsgemäßen Systems 1 geführt. An dem Handgriff 5 ist ein erstes Ende 9 des drahtartiges Elements 3 fixiert, während das zweite Ende 10 lösbar an dem Handgriff 5 befestigt ist. Zum Abwerfen des medizinischen Implantats 2 von dem erfindungsgemäßen System 1 wird die lösbare Verbindung zwischen Handgriff 5 und zweitem Ende 10 des drahtartigen Element 3 gelöst und das drahtartige Element mittels des Handgriffs 5 und dem daran fixierten ersten Ende 9 aus dem doppellumigen Führungsschlauch 4 gezogen, bis das drahtartige Element 3 nicht mehr in die Verbindungseinrichtung 8 des medizinischen Implantat 2 eingreift. Nachfolgend kann der doppellumige Führungsschlauch 4 aus dem Implantationskatheter 15 gezogen werden und abschließend wird der Implantationskatheter 15 aus dem menschlichen oder tierischen Körper entfernt.

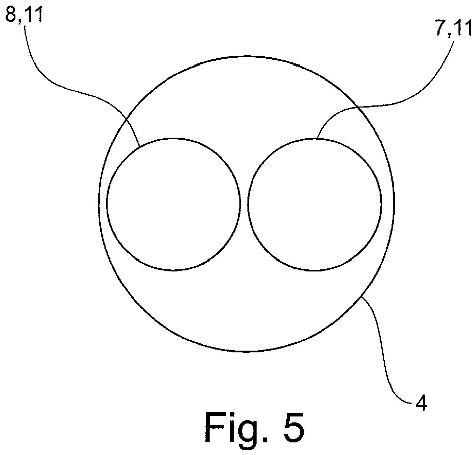

Figur 5 zeigt eine Querschnittsansicht eines doppellumigen Führungsschlauchs 4 eines erfindungsgemäßen Systems 1. Gemäß dem in Figur 5 dargestellten Ausführungsbeispiel umfasst der doppellumige Führungsschlauch 4 zwei innenliegende schlauchartige Elemente 11, welche das erste Lumen 6 und das zweite Lumen 7 des doppellumigen Führungsschlauchs 4 bilden. Die zwei innenliegenden schlauchartigen Elemente 11 sind beispielsweise jeweils als Edelstahlwendel ausgebildet.

### Bezugszeichenliste

- 1: System
- 2: medizinisches Implantat
- 3: drahtartiges Element
- 4: doppellumiger Führungsschlauch
- 5: Handgriff
- 6: erstes Lumen
- 7: zweites Lumen
- 8: Verbindungseinrichtung (medizinisches Implantat)
- 9: erstes Ende (drahtartiges Element)
- 10: zweites Ende (drahtartiges Element)
- 11: innenliegende schlauchartige Elemente
- 12: Nippel
- 13: Ausnehmung
- 14: Klemmmechanismus
- 15: Implantationskatheter

## Patentansprüche

1. System (1) zur Implantation eines medizinischen Implantats (2) im menschlichen oder tierischen Körper, umfassend:
ein drahtartiges Element (3) zur Verbindung des Systems (1) mit dem medizinischen Implantat (2),
einen doppellumigen Führungsschlauch (4) für das drahtartige Element (3), und
einen Handgriff (5) zur Handhabung des Systems (1),
wobei das medizinische Implantat (2) am proximalen Ende des Führungsschlauchs (4) angeordnet ist und der Handgriff (5) am distalen Ende des Führungsschlauchs (4), und wobei das drahtartige Element (3) mit einem ersten Ende (9) am Handgriff (5) fixiert ist, durch ein erstes Lumen (6) des doppellumigen Führungsschlauchs (4) zu dem medizinischen Implantat (2) geführt ist, mit einer Verbindungseinrichtung (8) des medizinischen Implantats (2) eingreift und durch ein zweites Lumen (7) des doppellumigen Führungsschlauchs (4) zum Handgriff (5) geführt ist und am Handgriff (5) mit einem zweiten Ende (10) lösbar fixiert ist,
**dadurch gekennzeichnet, dass**
der Handgriff (5) einen Klemmmechanismus (14) für das zweite Ende (10) des drahtartigen Elements (3), zur lösbaren Fixierung des zweiten Endes (10) des drahtartigen Elements (3) am Handgriff (5), und eine Sicherung gegen ein unbeabsichtigtes Betätigen des Klemmmechanismus (14) umfasst.

2. System (1) nach Anspruch 1,
wobei das drahtartige Element (3) aus einem Metall, insbesondere Edelstahl, besteht.

3. System (1) nach Anspruch 1 oder Anspruch 2,
wobei das drahtartige Element (3) einen Durchmesser von 0,1 mm bis 0,5 mm, insbesondere 0,2 mm, aufweist.

4. System (1) nach einem der Ansprüche 1 bis 3,
wobei der doppellumige Führungsschlauch (4) zumindest teilweise aus einem Kunststoff besteht.

5. System (1) nach einem der Ansprüche 1 bis 4,
wobei der doppellumige Führungsschlauch (4) einen Durchmesser von 1,0 mm bis 2,0 mm, insbesondere 1,76 mm, aufweist.

6. System (1) nach einem der Ansprüche 1 bis 5,
wobei der doppellumige Führungsschlauch (4) zwei innenliegende schlauchartige Elemente (11) umfasst, welche das erste Lumen (6) und das zweite Lumen (7) des doppellumigen Führungsschlauch (4) bilden.

7. System (1) nach Anspruch 6,
wobei die zwei innenliegenden schlauchartigen Elemente (11) jeweils als Edelstahlwendel ausgebildet sind.

8. System (1) nach einem der Ansprüche 1 bis 7,
wobei die Verbindungseinrichtung (8) des medizinischen Implantats (2) als Öse oder Schlaufe ausgebildet ist.

9. System (1) nach einem der Ansprüche 1 bis 8,
wobei das erste Ende (9) des drahtartigen Elements (3) einen Nippel (12) umfasst, welcher in einer entsprechenden Ausnehmung (13) des Handgriffs (5) fixierbar ist.

10. System (1) nach einem der Ansprüche 1 bis 9,
ferner umfassend einen Implantationskatheter (15) zur Führung des doppellumigen Führungsschlauchs (4) und des damit verbundenen medizinischen Implantats (2) zur Implantationsstelle im menschlichen oder tierischen Körper.

## Claims

1. System (1) for implanting a medical implant (2) in the human or animal body, comprising:
a wire-like element (3) for connecting the system (1) with the medical implant (2),
a double-lumen guiding tube (4) for the wire-like element (3), and
a handle (5) for operating the system (1),
wherein the medical implant (2) is located at the proximal end of the guiding tube (4) and the handle (5) at the distal end of the guiding tube (4), and wherein the wire-like element (3) is fixed with a first end (9) at the handle (5), guided through a first lumen (6) of the double-lumen guiding tube (4) to the medical implant (2), engaging connecting means (8) of the medical implant (2) and guided through a second lumen (7) of the double-lumen guiding tube (4) to the handle (5) and is detachable fixed to the handle (5) with a second end (10),
**characterized in that**
the handle (5) comprises clamping means (14) for the second end (10) of the wire-like element (3), for detachably fixing the second end (10) of the wire-like element (3) at the handle (5), and comprising a safeguard against unintentionally operating of the clamping means (14).

2. System (1) according to claim 1,
wherein the wire-like element (3) is made of metal, particularly of stainless steel.

3. System (1) according to claim 1 or claim 2,
wherein the wire-like element (3) has a diameter between 0,1 mm and 0,5 mm, particularly 0,2 mm.

4. System (1) according to one of claims 1 to 3,
wherein the double-lumen guiding tube (4) is at least partially made of plastics.

5. System (1) according to one of claims 1 to 4,
wherein the double-lumen guiding tube (4) has a diameter between 1,0 mm and 2,0 mm, particularly 1,76 mm.

6. System (1) according to one of claims 1 to 5,
wherein the double-lumen guiding tube (4) comprises two internal tube-like elements (11), which are the first lumen (6) and the second lumen (7) of the double-lumen guiding tube (4).

7. System (1) according to claim 6,
wherein the two internal tube-like elements (11) are each built as a steel spiral.

8. System according to one of claims 1 to 7,
wherein the connecting means (8) of the medical implant (2) is an eyelet or loop.

9. System (1) according to one of claims 1 to 8,
wherein the first end (9) of the wire-like element (3) comprises a fitting (12), which can be fixed in a corresponding recess (13) of handle (5).

10. System according to one of claims 1 to 9,
further comprising an implantation catheter (15) for guiding the double-lumen guiding tube (4) and the medical implant (2) connected thereto, to the implantation site in the human or animal body.

## Revendications

1. Système (1) d'implantation d'un implant médical (2) dans le corps humain ou animal, comprenant :
un élément filaire (3) permettant de connecter le système (1) à l'implant médical (2),
un tube de guidage à double lumière (4) pour l'élément filaire (3), et
une poignée (5) permettant de faire fonctionner le système (1),
l'implant médical (2) étant situé au niveau de l'extrémité proximale du tube de guidage (4) et la poignée (5) au niveau de l'extrémité distale du tube de guidage (4), et l'élément filaire (3) étant fixé avec une première extrémité (9) au niveau de la poignée (5), guidé à travers une première lumière (6) du tube de guidage à double lumière (4) vers l'implant médical (2), mettant en prise des moyens de connexion (8) de l'implant médical (2) et guidé à travers une deuxième lumière (7) du tube de guidage à double lumière (4) vers la poignée (5) et étant fixé amovible à la poignée (5) avec une seconde extrémité (10),
**caractérisé en ce que**
la poignée (5) comprend des moyens de serrage (14) pour la deuxième extrémité (10) de l'élément filaire (3), permettant de fixer de manière amovible la deuxième extrémité (10) de l'élément filaire (3) au niveau de la poignée (5), et comprenant une protection contre le fonctionnement involontaire du moyen de serrage (14).

2. Système (1) selon la revendication 1,
l'élément filaire (3) étant constitué de métal, en particulier d'acier inoxydable.

3. Système (1) selon la revendication 1 ou la revendication 2,
l'élément filaire (3) présentant un diamètre compris entre 0,1 mm et 0,5 mm, en particulier 0,2 mm.

4. Système (1) selon l'une des revendications 1 à 3,
le tube de guidage à double lumière (4) étant au moins partiellement constitué de plastique.

5. Système (1) selon l'une des revendications 1 à 4,
le tube de guidage à double lumière (4) présentant un diamètre compris entre 1,0 mm et 2,0 mm, en particulier 1,76 mm.

6. Système (1) selon l'une des revendications 1 à 5,
le tube de guidage à double lumière (4) comprenant deux éléments internes tubulaires (11), qui représentent la première lumière (6) et la deuxième lumière (7) du tube de guidage à double lumière (4).

7. Système (1) selon la revendication 6,
les deux éléments internes tubulaires (11) étant chacun construits sous la forme d'une spirale en acier.

8. Système selon l'une des revendications 1 à 7,
le moyen de connexion (8) de l'implant médical (2) étant un oeillet ou une boucle.

9. Système (1) selon l'une des revendications 1 à 8,
la première extrémité (9) de l'élément filaire (3) comprenant un raccord (12), qui peut être fixé dans un évidement correspondant (13) de la poignée (5).

10. Système selon l'une des revendications 1 à 9,
comprenant en outre un cathéter d'implantation (15) permettant de guider le tube de guidage à double lumière (4) et l'implant médical (2) qui y est connecté, vers le site d'implantation dans le corps humain ou animal.
